**Europäisches Patentamt**

**European Patent Office**

**Office européen des brevets**

(11) Publication number : **0 339 465 B1**

# EUROPEAN PATENT SPECIFICATION

(45) Date of publication of patent specification :
**09.09.92 Bulletin 92/37**

(21) Application number : **89107040.1**

(22) Date of filing : **19.04.89**

(51) Int. Cl.$^5$ : **A61K 31/545,** A61K 31/715, C08B 37/00, // (A61K31/715, 31:545)

(54) **Antibacterial composition for oral administration.**

(30) Priority : **19.04.88 JP 94543/88**

(43) Date of publication of application :
**02.11.89 Bulletin 89/44**

(45) Publication of the grant of the patent :
**09.09.92 Bulletin 92/37**

(84) Designated Contracting States :
**AT BE CH DE ES FR GB IT LI NL SE**

(56) References cited :
**EP-A- 0 163 433**

(73) Proprietor : **MEIJI SEIKA KAISHA LTD.**
**4-16 Kyobashi 2-chome**
**Chuo-ku Tokyo 104 (JP)**

(72) Inventor : **Kikkoji, Toshihiro Meiji Seika Kaisha, Ltd.**
**Pharm. Dev. Lab. 580, Horikawa-cho Saiwai-ku**
**Kawasaki-shi Kanagawa (JP)**
Inventor : **Kobayashi, Toshiyuki Meiji Seika Kaisha, Ltd.**
**Pharm. Dev. Lab. 580, Horikawa-cho Saiwai-ku**
**Kawasaki-shi Kanagawa (JP)**
Inventor : **Sato, Toyomi Meiji Seika Kaisha, Ltd.**
**Pharm. Dev. Lab. 580, Horikawa-cho Saiwai-ku**
**Kawasaki-shi Kanagawa (JP)**
Inventor : **Watanabe, Koji Meiji Seika Kaisha, Ltd.**
**Pharm. Dev. Lab. 580, Horikawa-cho Saiwai-ku**
**Kawasaki-shi Kanagawa (JP)**
Inventor : **Nishizawa, Kenji Meiji Seika Kaisha, Ltd.**
**Pharm. Dev. Lab. 580, Horikawa-cho Saiwai-ku**
**Kawasaki-shi Kanagawa (JP)**

(74) Representative : **WILHELMS, KILIAN & PARTNER Patentanwälte**
**Eduard-Schmid-Strasse 2**
**W-8000 München 90 (DE)**

## Description

FIELD OF THE INVENTION

This invention relates to an antibacterial composition for oral administration containing pivaloyloxymethyl-(6R,7R)-7[(Z)-2-(2-aminothiazol-4-yl)-2-methoxyiminoacetamide]-3-[(Z)-2-(4-methylthiazol-5-yl)ethenyl]cephem-4-carboxylate (hereinafter referred to as the substance ME1207), β-cyclodextrin and a pharmaceutically acceptable carrier.

BACKGROUND OF THE INVENTION

The substance ME1207 is a cephem antibiotic for internal use and is a prodrug which is absorbed through the intestine when orally administered and hydrolyzed by esterase present at the intestinal wall to thereby give (6R,7R)-7[(Z)-2-(2-aminothiazol-4-yl)-2-methoxyiminoacetamide]-3-[(Z)-2-(4-methylthiazol-5-yl)ethenyl]cephem-4-carboxylic acid (hereinafter referred to as the substance ME1206) having an antibacterial activity. This substance ME1206 has a wide antibacterial spectrum against gram-positive and gram-negative bacteria and is highly useful in the treatment and prevention of bacterial infectious diseases (cf. EP-A-175610).

However, the substance ME1207 has a serious disadvantage that, when orally administered to a hungry recipient in the form of a preparation for oral administration, for example, tablets, capsules, powder, granules, fine granules or dry syrup, the substance ME1207 shows considerably low absorbability compared with the case when administered after meals. It is generally known that a meal is one of factors that affect the absorbability of a drug through the digestive tract (cf. Y. Nakai and M. Hanano, "Seizaigaku (Pharmaceutics)", Nanzando). Namely, meals prolong the gastric emptying time and the absorption site passage time of a drug and enhance the acid secretion. Thus, the absorption of the drug is promoted or suppressed depending on the physicochemical and biopharmaceutical properties thereof.

On the other hand, it is known that a fat-soluble cephalosporin compound for oral administration is hardly soluble in water and has an oil/water partition ratio of 100 to 1,000 so that it shows a low absorbability through the digestive tract. There are reports with respect to the improvement of the absorbability of the fat-soluble cephalosporin that the addition of cyclodextrins, in particular, α-cyclodextrin in an amount of approximately 10 to 70 wt% based on the weight of cephalosporin can elevate the water-solubility thereof [cf. JP-A-60-233012 and 62-30713 (the term "JP-A" as used herein means "an unexamined published Japanese patent application") which corresponds to U.S. Patent 4,616,008 or EP-A-163433].

SUMMARY OF THE INVENTION

An object of the present invention is to enable a cephalosporin compound having an oil/water partition ratio lower than 100 to be absorbed through the degestive tract when orally administered to a hungry recipient.

In order to achieve the above object, we have conducted extensive studies from the pharmaceutical view point and consequently obtained the following findings.

(1) When the substance ME1207 is administered to a hungry patient, it shows a poor dispersibility in the empty stomach, which makes its absorbability low.

(2) The dispersibility of the substance ME1207 in the empty stomach and the digestive tract can be improved by adding, to the substance ME1207 having an oil/water partition ratio of about 40 to 60, cyclodextrins, particularly β-cyclodextrin which is hardly soluble in water compared with α-cyclodextrin and γ-cyclodextrin, in an amount 1 to 16 parts by weight, preferably 1 to 3 parts by weight, per part by weight of the substance ME1207. Thus, the absorbability of the substance ME1207 can be elevated.

DETAILED DESCRIPTION OF THE INVENTION

In order to improve dispersibility and absorbability of the substance ME1207, the composition of the present invention may further contain, for example, an ionic surfactant such as sodium di-2-ethylhexylsulfosuccinate (OTP-100 NIKKOL®) or sodium lauryl sulfate, a nonionic surfactant such as polyoxyethylene hydrogenated castor oil (HCO-60 NIKKOL®), polyoxyethylene alkyl ether (BL-9EX NIKKOL®) or polyethylene glycol fatty acid ester (MYS-40 NIKKOL®) (the trade names of surfactants as listed above are all products manufactured by Nikko Chemicals) or lecithin which is the major constituent of a biomembrane and has surface activity. These surfactants are added in an amount of approximately 1 to 100 wt%, preferably 2 to 20 wt%, based on the weight of the substance ME1207. Among these, polyethylene glycol fatty acid ester is preferably used in an amount of 2 to 4 wt% based on the weight of the substance ME1207. The substance ME1207 is unstable in an aqueous solution of a pH value of 6 or above. Accordingly, a substance capable of the lowering pH value may be further added with the purpose of preventing the decomposition of the substance ME1207 and improving the absorbability of the same. Examples of these substance include an organic acid such as maleic acid, tartaric acid, citric acid, malic acid or ascorbic acid. These substance may be added in an amount of approximately 10 to 300 wt%, preferably 50 to 150 wt%, based on the weight of the substance ME1207.

The composition of the present invention may further contain a pharmaceutically acceptable binder, vehicle, disintegrating agent, edulcorant, perfume, colorant and/or lubricant. The composition thus obtained may be formulated into, for example, tablets, capsules, powders, granules, fine granules or dry syrups in a conventional manner.

The substance ME1207 thus formulated is administered to a patient in a dose of approximately 50 to 500 mg two or three times per day.

The following Examples and Reference Examples will be given to further illustrate the present invention, but are not construed to limit the scope of the present invention.

## EXAMPLE 1

130 g of the substance ME1207, 260 g of β-cyclodextrin and 5 g of hydroxypropylmethylcellulose were homogeneously mixed and subjected to wet granulation in a conventional manner. Separately, 60 g of lactose and 6 g of light silicic acid anhydride were homogenized together. These two powders were mixed and 6 g of magnesium stearate was further added thereto. The thus-obtained mixture was tableted in a conventional manner to thereby give tablets each having the following composition.

```
substance ME1207                          130 mg

β-cyclodextrin                            260 mg

hydroxypropylmethylcellulose                5 mg

lactose                                    60 mg

light silicic acid anhydride                6 mg

magnesium stearate                          6 mg

                             total        467 mg
```

## EXAMPLE 2

130 g of the substance ME1207, 130 g of β-cyclodextrin and 5 g of hydroxypropylmethylcellulose were homogeneously mixed and subjected to wet granulation in a conventional manner. Separately, 10 g of hydroxypropylcellulose of a low degree of substitution and 150 g of citric acid were homogenized together. These two powders were mixed and 6 g of magnesium stearate was further added thereto. The resulting mixture was tableted in a conventional manner to thereby give tablets each having the following composition.

```
substance ME1207                          130 mg

β-cyclodextrin                            130 mg

hydroxypropylmethylcellulose                5 mg

hydroxypropylcellulose of a low
degree of substitution                     10 mg
```

```
citric acid                           150 mg

magnesium stearate                      6 mg

                          total       431 mg
```

EXAMPLE 3

130 g of the substance ME1207, 130 g of β-cyclodextrin, 5 g of hydroxypropylmethylcellulose and 5 g of sodium di-2-ethylhexylsulfosuccinate were homogeneously mixed and subjected to wet granulation in a conventional manner. Separately, 40 g of hydroxypropylcellulose of a low degree of substitution and 6 g of silicic acid anhydride were homogenized together. These two powders were mixed and 6 g of magnesium stearate was further added thereto. The thus-obtained mixture was tableted in a conventional manner to thereby give tablets each having the following composition.

```
substance ME1207                      130 mg

β-cyclodextrin                        130 mg

hydroxypropylmethylcellulose            5 mg

sodium di-2-ethylhexylsulfosuccinate  5 mg

hydroxypropylcellulose of a low
degree of substitution                 40 mg

silicic acid anhydride                  6 mg

magnesium stearate                      6 mg

                          total       322 mg
```

EXAMPLE 4

130 g of the substance ME1207, 200 g of β-cyclodextrin, 26 g of lecithin and 50 g of corn starch were homogeneously mixed and subjected to wet granulation in a conventional manner. Separately, 50 g of lactose and 70 g of succinic acid were homogenized together. These two powders were mixed and 6 g of magnesium stearate was further added thereto. The thus-obtained mixture was tableted in a conventional manner to thereby give tablets each having the following composition.

```
substance ME1207                      130 mg

β-cyclodextrin                        200 mg

lecithin                               26 mg

corn starch                            50 mg

lactose                                50 mg

succinic acid                          70 mg

magnesium stearate                      6 mg

                          total       532 mg
```

4

EXAMPLE 5

130 g of the substance ME1207, 130 g of β-cyclodextrin, 5 g of hydroxypropylmethylcellulose and 7 g of polyoxyethylene hydrogenated castor oil (HCO-60 NIKKOL®) were homogeneously mixed and subjected to wet granulation in a conventional manner. 6 g of magnesium stearate was further added thereto. The resulting mixture was encapsulated in a conventional manner to thereby give capsules each having the following composition.

```
substance ME1207                          130 mg

β-cyclodextrin                            130 mg

hydroxypropylmethylcellulose                5 mg

polyoxyethylene hydrogenaed castor oil
(HCO-60 NIKKOL®)                            7 mg

magnesium stearate                          6 mg

                                total     278 mg
```

EXAMPLE 6

130 g of the substance ME1207, 260 g of β-cyclodextrin, 5 g of hydroxypropylmethylcellulose, 103 g of D-mannitol, 195 g of malic acid and 7 g of polyethylene glycol fatty acid ester (MYS-40 NIKKOL®) were homogeneously mixed. The resulting powder was subjected to wet granulation in a conventional manner and then formulated into fine granules which had the following composition per 700 mg.

```
substance ME1207                          130 mg

β-cyclodextrin                            260 mg

hydroxypropylmethylcellulose                5 mg

D-mannitol                                103 mg

malic acid                                195 mg

polyethylene glycol fatty acid
ester (MYS-40 NIKKOL®)                      7 mg

                                total     700 mg
```

REFERENCE EXAMPLE 1

130 g of the substance ME1207 and 5 g of hydroxypropylmethylcellulose were homogeneously mixed and subjected to wet granulation in a conventional manner. Separately, 60 g of lactose and 6 g of light silicic acid anhydride were homogenized together. These two powders were mixed and 6 g of magnesium stearate was further added thereto. The thus-obtained mixture was tableted in a conventional manner to thereby give tablets each having the following composition.

| substance ME1207 | 130 mg |
|---|---|
| hydroxypropylmethylcellulose | 5 mg |
| lactose | 60 mg |
| light silicic acid anhydride | 6 mg |
| magnesium stearate | 6 mg |
| total | 207 mg |

REFERENCE EXAMPLE 2

130 g of the substance ME1207 and 5 g of hydroxypropylmethylcellulose were homogeneously mixed and subjected to wet granulation in a conventional manner. Separately, 10 g of hydroxypropylcellulose of a low degree of substitution and 150 g of citric acid were homogenized together. These two powders were mixed and 6 g of magnesium stearate was further added thereto. The mixture thus obtained was tableted in a conventional manner to thereby give tablets each having the following composition.

| substance ME1207 | 130 mg |
|---|---|
| hydroxypropylmethylcellulose | 5 mg |
| hydroxypropylcellulose of a low degree of substitution | 10 mg |
| citric acid | 150 mg |
| magnesium stearate | 6 mg |
| total | 301 mg |

REFERENCE EXAMPLE 3

130 g of the substance ME1207, 5 g of hydroxypropylmethylcellulose and 7 g of polyoxyethylene hydrogenated castor oil (HCO-60 NIKKOL®) were homogeneously mixed and subjected to wet granulation in a conventional manner. 6 g of magnesium stearate was further added thereto. The resulting mixture was encapsulated in a conventional manner to thereby give capsules each having the following composition.

| substance ME1207 | 130 mg |
|---|---|
| hydroxypropylmethylcellulose | 5 mg |
| polyoxyethylene hydrogenated castor oil (HCO-60 NIKKOL®) | 7 mg |
| magnesium stearate | 6 mg |
| total | 148 mg |

TEST EXAMPLE 1

The dispersibility of each preparation obtained in the above Examples 1 through 6 and Reference Examples 1 through 3 was evaluated in the following manner.

Test method:

A 200 ml portion of water was charged in each of nine 200 ml Erlenmeyer flasks and maintained at 37°C. Each drug was added to each flask and allowed to stand for 30 minutes. After shaking ten times, it was allowed to stand for additional ten minutes. Then, 1 ml of the suspension 1 cm below its surface was collected with a whole pipette. To the resulting suspension, 1 ml of acetonitrile was added and the drug was completely dissolved. The concentration of the substance ME1207 in the sample was determined by high performance liquid chromatography. When the content of the β-cyclodextrin in the preparation was less than two times as much as the substance ME1207, β-cyclodextrin was preliminarily added to 200 ml of the water so as to equalize the β-cyclodextrin concentrations of all samples.

Table 1 shows the results of the determination of the substance ME1207 concentrations in the suspensions.

## Table 1

| Sample | Substance ME1207 concentation in suspension (μg/ml) |
|---|---|
| Example 1 | 473.6 ± 35.1 |
| Example 2 | 293.0 ± 22.2 |
| Example 3 | 393.7 ± 31.3 |
| Example 4 | 381.1 ± 28.0 |
| Example 5 | 318.9 ± 39.7 |
| Example 6 | 532.5 ± 43.5 |
| Reference Example 1 | 73.2 ± 11.2 |
| Reference Example 2 | 68.5 ± 9 3 |
| Reference Example 3 | 35.4 ± 6.8 |

TEST EXAMPLE 2

The absorbability of the composition of the invention was evaluated by the following method.

Test method:

Two tablets obtained in the above Example 1 and Reference Example 3 and 1.4 g of granules obtained in example 6, each containing 260 mg of the substance ME1207, were orally administered to each of 6 female beagles weighing approximately 10 kg together with 30 ml of water. 0.25, 0.5, 1, 2, 4, 6 and 8 hours after the administration, the concentration of the substance ME1207 in the plasma of each animal was determined by high performance liquid chromatography and the area under the plasma drug concentration curve (AUC) was calculated by the trapezoidal method. These three preparations were administered after fasting for 24 hours and 30 minutes after meals according to the protocol defined in Table 2. A rest time of two weeks was provided between test periods.

## Table 2: Administration schedule

| Animal No. | 1st | 2nd | 3rd | 4th | 5th | 6th |
|---|---|---|---|---|---|---|
| 1 | a | (b) | c | (a) | b | (c) |
| 2 | (a) | b | (c) | a | (b) | c |
| 3 | b | (c) | a | (b) | c | (a) |
| 4 | (b) | c | (a) | b | (c) | a |
| 5 | c | (a) | b | (c) | a | (b) |
| 6 | (c) | a | (b) | c | (a) | b |

Note:  a: Preparation of Example 1

b: Preparation of Example 6

c: Preparation of Reference Example 3

( ): Administered at a hungry state

Table 3 shows the concentrations of the substance ME1207 in plasma and AUC in the case of the administration at a hungry state, and Table 4 shows those in the case of the administration after meals.

## Table 3

| Sample | Substance ME1207 concentration ($\mu$g/ml) | | | | | | | AUC ($\mu$g/ml·hr) |
|---|---|---|---|---|---|---|---|---|
| | 0.25 (hr) | 0.50 (hr) | 1.00 (hr) | 2.00 (hr) | 4.00 (hr) | 6.00 (hr) | 8.00 (hr) | |
| Example 1 | 0.71 (0.31) | 1.73 (0.26) | 2.34 (0.19) | 1.79 (0.08) | 0.40 (0.11) | 0.22 (0.05) | 0.10 (0.05) | 6.59 (0.68) |
| Example 6 | 0.91 (0.35) | 1.97 (0.37) | 2.55 (0.38) | 1.96 (0.45) | 0.52 (0.14) | 0.29 (0.11) | 0.14 (0.04) | 7.58 (1.28) |
| Reference Example 3 | 0.03 (0.02) | 0.14 (0.06) | 0.05 (0.01) | 0.02 (0.01) | 0.01 (0.00) | 0.00 (0.00) | 0.00 (0.00) | 0.15 (0.04) |

Note:  The value of upper line is a mean value, while the parenthetic value of lower line is S.E.

## Table 4

| Sample | Substance ME1207 concentration (μg/ml) | | | | | | | AUC (μg/ml·hr) |
|---|---|---|---|---|---|---|---|---|
| | 0.25 (hr) | 0.50 (hr) | 1.00 (hr) | 2.00 (hr) | 4.00 (hr) | 6.00 (hr) | 8.00 (hr) | |
| Example 1 | 0.52 (0.08) | 1.61 (0.21) | 2.49 (0.18) | 1.91 (0.14) | 0.41 (0.06) | 0.30 (0.04) | 0.10 (0.03) | 6.97 (0.60) |
| Example 6 | 0.60 (0.18) | 1.50 (0.27) | 2.39 (0.18) | 2.20 (0.20) | 0.40 (0.08) | 0.28 (0.07) | 0.10 (0.03) | 7.25 (0.76) |
| Reference Example 3 | 1.06 (0.08) | 2.10 (0.18) | 2.46 (0.08) | 1.73 (0.06) | 0.45 (0.09) | 0.23 (0.06) | 0.08 (0.03) | 6.91 (0.42) |

Note: The value of upper line is a mean value, while the parenthetic value of lower line is S.E.

As is apparent from the results shown in Tables 3 and 4, the compositions according to the present invention provide the elevated absorbability of the substance ME1207 in the case of oral administration to a hungry recipient to a level comparable to that in the case of administration after meals. Thus, the availability of the substance ME1207 is highly improved.

## Claims

### Claims for the following Contracting States : AT, BE, CH, DE, FR, GB, IT, LI, NL, SE

1. An antibacterial composition for oral administration comprising pivaloyloxymethyl-(6R,7R)-7[(Z)-2-(2-aminothiazol-4-yl)-2-methoxyiminoacetamide]-3-[(Z)-2-(4-methylthiazol-5-yl)ethenyl]cephem-4-carboxylate (hereinafter referred to as the substance ME1207), β-cyclodextrin and a pharmaceutically acceptable carrier.

2. The antibacterial composition according to claim 1, wherein said β-cyclodextrin is contained in an amount of 1 to 16 parts by weight per part by weight of the substance ME1207.

3. The antibacterial composition according to claim 1, wherein said β-cyclodextrin is contained in an amount of 1 to 3 parts by weight per part by weight of the substance ME1207.

4. The antibacterial composition according to claim 1, which contains a surfactant selected from sodium di-2-ethylhexylsulfosuccinate, sodium lauryl sulfate, polyoxytethylene hydrogenated castor oil, polyoxyethylene alkyl ether, polyethylene glycol fatty acid ester and lecithin.

5. The antibacterial composition according to claim 4, wherein said surfactant is contained in an amount of approximately 1-100 wt% based on the weight of the substance ME1207.

6. The antibacterial composition according to claim 1, which contains a substance capable of lowering pH value selected from maleic acid, tartaric acid, citric acid, succinic acid, malic acid and ascorbic acid.

7. The antibacterial composition according to claim 6, wherein said substance is contained in an amount of approximately 10 to 300 wt% based on the weight of the substance ME1207.

### Claims for the following Contracting State : ES

1. The use of a composition for oral administration comprising pivaloyloxymethyl-(6R,7R)-7[(Z)-2-(2-aminothiazol-4-yl)-2-methoxyimino-acetamide]-3-[(Z)-2-(4-methylthiazol-5-yl)ethenyl]cephem-4-carboxylate, β-cyclodextrin and a pharmaceutically aceptable carrier for the manufacture of a medicament for the treatment and/or prevention of bacterial infectious diseases.

2. The use of a composition according to claim 1, wherein said β-cyclodextrin is contained in an amount of 1 to 16 parts by weight per part by weight of the substance ME1207.

3. The use of a composition according to claim 1, wherein said β-cyclodextrin is contained in an amount of 1 to 3 parts by weight per part by weight of the substance ME1207.

4. The use of a composition according to claim 1, which contains a surfactant selected from sodium di-2-ethylhexylsulfosuccinate, sodium lauryl sulfate, polyoxytethylene hydrogenated castor oil, polyoxyethylene alkyl ether, polyethylene glycol fatty acid ester and lecithin.

5. The use of a composition according to claim 4, wherein said surfactant is contained in an amount of approximately 1-100 wt% based on the weight of the substance ME1207.

6. The use of a composition according to claim 1, which contains a substance capable of lowering pH value selected from maleic acid, tartaric acid, citric acid, succinic acid, malic acid and ascorbic acid.

7. The use of a composition according to claim 6, wherein said substance is contained in an amount of approximately 10 to 300 wt% based on the weight of the substance ME1207.

## Patentansprüche

### Patentansprüche für folgende Vertragsstaaten : AT, BE, CH, DE, FR, GB, IT, LI, NL, SE

1. Antibakterielle Zusammensetzung zur oralen Verabreichung mit Gehalt an Pivaloyloxymethyl-(6R,7R)-7[(Z)-2-(2-aminothiazol-4-yl)-2-methoxyiminoacetamid]-3-[(Z)-2-(4-methylthiazol-5-yl)ethenyl]cephem-4-carboxylat (im folgenden als Substanz ME1207 bezeichnet), β-Cyklodextrin und einem pharmazeutisch verträglichen Träger.

2. Antibakterielle Zusammensetzung nach Anspruch 1, wobei das β-Cyklodextrin in Mengen von 1 bis 16 Gewichtsteilen je Gewichtsteil der Substanz ME1207 enthalten ist.

3. Antibakterielle Zusammensetzung nach Anspruch 1, wobei das β-Cyklodextrin in Mengen von 1 bis 3 Gewichtsteilen je Gewichtsteil der Substanz ME1207 enthalten ist.

4. Antibakterielle Zusammensetzung nach Anspruch 1, die eines der oberflächenaktiven Mittel Natrium-di-2-ethylhexylsulfosuccinat, Natriumlaurylsulfat, Polyoxyethylen-hydriertes Castoröl, Polyoxyethylenalkyläther, Polyethylenglykolfettsäureester und/oder Lecithin enthält.

5. Antibakterielle Zusammensetzung nach Anspruch 4, wobei das oberflächenaktive Mittel in Mengen von etwa 1 bis 100 Gew.-%, bezogen auf das Gewicht der Substanz ME1207, enthalten ist.

6. Antibakterielle Zusammensetzung nach Anspruch 1, die eine Substanz zur Erniedrigung des pH-Wertes enthält, nämlich Maleinsäure, Weinsäure, Zitronensäure, Succinsäure, Maleinsäure und/oder Ascorbinsäure.

7. Antibakterielle Zusammensetzung nach Anspruch 6, wobei die Substanz in Mengen von etwa 10 bis 300 Gew.-%, bezogen auf das Gewicht der Substanz ME1207, enthalten ist.

### Patentansprüche für folgenden Vertragsstaat : ES

1. Verwendung einer Zusammensetzung zur oralen Verabreichung zur Herstellung eines Arzneimittels zur Behandlung und/oder Verhinderung bakteriell-infektiöser Erkrankungen, diePivaloyloxymethyl-(6R,7R)-7[(Z)-2-(2-aminothiazol-4-yl)-2-methoxyiminoacetamid]-3-[(Z)-2-(4-methylthiazol-5-yl)ethenyl]cephem-4-carboxylat, β-Cyklodextrin und einen pharmazeutisch verträglichen Träger enthält.

2. Verwendung einer Zusammensetzung nach Anspruch 1, wobei das β-Cyklodextrin in Mengen von 1 bis 16 Gewichtsteilen je Gewichtsteil der Substanz ME1207 enthalten ist.

**3.** Verwendung einer Zusammensetzung nach Anspruch 1, wobei das β-Cyklodextrin in Mengen von 1 bis 3 Gewichtsteilen je Gewichtsteil der Substanz ME1207 enthalten ist.

**4.** Verwendung einer Zusammensetzung nach Anspruch 1, die eines der oberflächenaktiven Mittel Natrium-di-2-ethylhexylsulfosuccinat, Natriumlaurylsulfat, Polyoxyethylen-hydriertes Castoröl, Polyoxyethylenalkyläther, Polyethylenglykolfettsäureester und/oder Lecithin enthält.

**5.** Verwendung einer Zusammensetzung nach Anspruch 4, wobei das oberflächenaktive Mittel in Mengen von etwa 1 bis 100 Gew.-%, bezogen auf das Gewicht der Substanz ME1207, enthalten ist.

**6.** Verwendung einer Zusammensetzung nach Anspruch 1, die eine Substanz zur Erniedrigung des pH-Wertes enthält, nämlich Maleinsäure, Weinsäure, Zitronensäure, Succinsäure, Maleinsäure und/oder Ascorbinsäure.

**7.** Verwendung einer Zusammensetzung nach Anspruch 6, wobei die Substanz in Mengen von etwa 10 bis 300 Gew.-%, bezogen auf das Gewicht der Substanz ME1207, enthalten ist.

**Revendications**

**Revendications pour les Etats contractants suivants : AT, BE, CH, DE, FR, GB, IT, LI, NL, SE**

**1.** Composition antibactérienne pour une administration orale, comprenant du pivaloyloxyméthyl-(6R,7R)-7[(Z)-2-(2-aminothiazol-4-yl)-2-méthoxyiminoacétamide]-3-[(Z)-2-(4-méthylthiazol-5-yl)éthényl] cephem-4-carboxylate (désignée ci-après comme la substance ME1207), une β-cyclodextrine et un support pharmaceutiquement acceptable.

**2.** Composition antibactérienne selon la revendication 1, dans laquelle ladite β-cyclodextrine est contenue selon une quantité de 1 à 16 parties en poids par partie en poids de la substance ME1207.

**3.** Composition antibactérienne selon la revendication 1, dans laquelle ladite β-cyclodextrine est contenue selon une quantité de 1 à 3 parties en poids par partie en poids de la substance ME1207.

**4.** Composition antibactérienne selon la revendication 1, qui contient un agent tensio-actif choisi parmi le di-2-éthylhexylsulfosuccinate de sodium, le lauryl-sulfate de sodium, l'huile de ricin hydrogénée du polyoxyéthylène, le polyoxyéthylène-alkyléther, l'ester d'acide gras du polyéthylène-glycol et la lécithine.

**5.** Composition antibactérienne selon la revendication 4, dans laquelle ledit agent tensio-actif est contenu selon une quantité de 1 à 100% en poids approximativement, basé sur le poids de la substance ME1207.

**6.** Composition antibactérienne selon la revendication 1, qui contient une substance capable d'abaisser la valeur du pH, choisie parmi l'acide maléique, l'acide tartrique, l'acide citrique, l'acide succinique, l'acide malique et l'acide ascorbique.

**7.** Composition antibactérienne d'une composition selon la revendication 6, dans laquelle ladite substance est contenue selon une quantité de 10 à 300% en poids approximativement, basé sur le poids de la substance ME1207.

**Revendications pour l'Etat contractant suivant : ES**

**1.** Utilisation d'une composition pour une administration orale, comprenant du pivaloyloxyméthyl-(6R,7R)-7[(Z)-2-(2-aminothiazol-4-yl)-2-méthoxyiminoacétamide]-3-[(Z)-2-(4-méthylthiazol-5-yl)éthényl] cephem-4-carboxylate, une β-cyclodextrine et un support pharmaceutiquement acceptable pour la fabrication d'un médicament pour le traitent et/ou la prévention de maladies infectieuses bactériennes.

**2.** Utilisation d'une composition selon la revendication 1, dans laquelle ladite β-cyclodextrine est contenue selon une quantité de 1 à 16 parties en poids par partie en poids de la substance ME1207.

**3.** Utilisation d'une composition selon la revendication 1, dans laquelle ladite β-cyclodextrine est contenue selon une quantité de 1 à 3 parties en poids par partie en poids de la substance ME1207.

4. Utilisation d'une composition selon la revendication 1, qui contient un agent tensio-actif choisi parmi le di-2-éthylhexylsulfosuccinate de sodium, le lauryl-sulfate de sodium, l'huile de ricin hydrogénée du polyoxyéthylène, le polyoxyéthylène-alkyléther, l'ester d'acide gras du polyéthylène-glycol et la lécithine.

5. Utilisation d'une composition selon la revendication 4, dans laquelle ledit agent tensio-actif est contenu selon une quantité de 1 à 100% en poids approximativement, basé sur le poids de la substance ME1207.

6. Utilisation d'une composition selon la revendication 1, qui contient une substance capable d'abaisser la valeur du pH, choisie parmi l'acide maléique, l'acide tartrique, l'acide citrique, l'acide succinique, l'acide malique et l'acide ascorbique.

7. Utilisation d'une composition selon la revendication 6, dans laquelle ladite substance est contenue selon une quantité de 10 à 300% en poids approximativement, basé sur le poids de la substance ME1207.